# EUROPÄISCHE PATENTANMELDUNG

(11) **EP 3 072 424 A1**
(43) Veröffentlichungstag der Anmeldung: **28.09.2016**
(21) Anmeldenummer: 16160905.2
(22) Anmeldetag: 17.03.2016
(51) Int. Cl.: A47J 27/088, A61L 11/00

(54) **VORRICHTUNG ZUM ERHITZEN, DESINFIZIEREN UND/ODER STERILISIEREN EINES GUTES UNTER ÜBERDRUCK**

(30) Priorität: 20.03.2015 AT 502252015
(71) Anmelder: Meteka GmbH, 8750 Judenburg (AT)
(72) Erfinder: Katschnig, Roland, 8750 Judenburg (AT); Weiermair, Günther, 8570 Judenburg (AT)
(74) Vertreter: Vinazzer, Edith

(57) **Zusammenfassung**

Vorrichtung zum Erhitzen, Desinfizieren und/oder Sterilisieren eines Gutes unter Überdruck durch Erhitzen des Gutes mittels Mikrowellen, mit einem in einer Behandlungskammer (1) auf einem Drehteller positionierbaren Behälter (2), in welchem sich das zu behandelnde Gut befindet und welcher mit einem Deckel (3) verriegelbar ist, welcher mit einer Öffnung versehen ist, durch welche Flüssigkeit in das Behälterinnere einbringbar ist, und mit einer Hubeinheit (5) mit einem Hubmotor (17), einer Gleitringaufnahme (18) und einem auf dem Deckel (3) aufsetzbaren kuppelförmigen Gleitring (19), wobei der Hubmotor (17), die Gleitringaufnahme (18) und der Gleitring (19) miteinander fest verbunden sind.

Auf der Hubeinheit (5) ist eine Anordnung aus einer an die Außenabmessungen des Deckels (3) angepassten Druckplatte (21) und einer oberhalb der Druckplatte (21) und von dieser in vertikaler Richtung beabstandeten, mit der Druckplatte (21) verbundenen Mitnehmerplatte (22) positioniert, wobei die Mitnehmerplatte (22) gegenüber dem Gleitring (19) oder der Gleitringaufnahme (18) drehbar ist und die Druckplatte (21) in vertikaler Richtung bewegbar ist, wobei bei ausgefahrener Hubeinheit (5) die Druckplatte (21) von der Dichtungsglocke (4) und dem Gleitring (19) auf den Behälterdeckel (3) gedrückt wird.

## Beschreibung

Die Erfindung betrifft eine Vorrichtung zum Erhitzen, Desinfizieren und/oder Sterilisieren eines Gutes unter Überdruck durch Erhitzen des Gutes mittels Mikrowellen, mit einem in einer Behandlungskammer auf einem Drehteller positionierbaren Behälter, in welchem sich das zu behandelnde Gut befindet und welcher mit einem Deckel verriegelbar ist, welcher mit einer Öffnung versehen ist, durch welche Flüssigkeit in das Behälterinnere einbringbar ist, und mit einer Hubeinheit mit einem Hubmotor, einer Gleitringaufnahme und einem auf dem Deckel aufsetzbaren kuppelförmigen Gleitring, wobei der Hubmotor, die Gleitringaufnahme und der Gleitring miteinander fest verbunden sind, sowie mit einer gegenüber der Gleitringaufnahme abgedichteten, jedoch drehbaren, auf dem Deckel aufsetzbaren Dichtungsglocke, welche über den Gleitring gestülpt ist.

Derartige Vorrichtungen zur Verwendung unter Umgebungsdruck sind bekannt und seit einigen Jahren vor allem zum Desinfizieren oder Sterilisieren im Einsatz. Eine ähnliche Vorrichtung ist aus dem österreichischen Patent Nr. 403007 bekannt.

Der während des automatisch ablaufenden Desinfektions- oder Sterilisationsvorganges im Inneren des Behälters während eines Behandlungszyklus entstehende Dampfdruck kann in besonderen Fällen derart groß werden, dass die Möglichkeit besteht, dass der Deckel an seinen Randbereichen nicht mehr optimal abdichtet.

Der Erfindung liegt somit die Aufgabe zu Grunde, bei einer Vorrichtung der eingangs genannten Art auch bei höherem Dampfdruck im Inneren des Behälters sicherzustellen, dass kein Dampf auf unerwünschte Weise aus dem Behälter in die Behandlungskammer austreten kann und dass der Dampfdruck aufrecht bleibt, um eine optimale Entkeimung sicherzustellen.

Gelöst wird die gestellte Aufgabe erfindungsgemäß dadurch, dass auf der Hubeinheit eine Anordnung aus einer an die Außenabmessungen des Deckels angepassten Druckplatte und einer oberhalb der Druckplatte und von dieser in vertikaler Richtung beabstandeten, mit der Druckplatte verbundenen Mitnehmerplatte positioniert ist, wobei die Mitnehmerplatte gegenüber dem Gleitring oder der Gleitringaufnahme drehbar ist und die Druckplatte in vertikaler Richtung bewegbar ist, wobei bei ausgefahrener Hubeinheit die Druckplatte von der Dichtungsglocke und dem Gleitring auf den Behälterdeckel gedrückt wird.

Die Erfindung gewährleistet eine optimale Abdichtung des Behälters gegenüber der Behandlungskammer, da der von der Druckplatte beaufschlagte Deckel eine gleichmäßige Verteilung des von innen auf den Deckel wirkenden Dampfdruckes gewährleistet. Die Anordnung aus Druckplatte und Mitnehmerplatte ist derart an der Hubeinheit angeordnet, dass die Druckplatte gegenüber dieser drehbar ist und in vertikaler Richtung bewegbar ist. Die Hubeinheit kann nach wie vor automatisch betätigt werden, da die Mitnehmerplatte die erforderliche Beweglichkeit der Anordnung sicherstellt und ein zentriertes Aufsetzen der Dichtungsglocke und des Gleitringes auf die Druckplatte gewährleistet ist.

Bei einer bevorzugten Ausführungsform der Erfindung sind die Druckplatte und die Mitnehmerplatte miteinander fest verbunden, wobei die Mitnehmerplatte gegenüber der Gleitringaufnahme drehbar und in vertikaler Richtung bewegbar ist und in der eingefahrenen Position der Hubeinheit auf dem Gleitring bzw. der Dichtungsglocke aufliegt. Bei dieser Ausführung bewegt sich beim Ausfahren der Hubeinheit die gesamte Anordnung aus Mitnehmerplatte und Druckplatte gegenüber der Hubeinheit in vertikaler Richtung.

Bevorzugt ist ferner die Druckplatte mit der Mitnehmerplatte auf einfache Weise über stegartig ausgebildete Mitnehmer fest verbunden.

Bei einer alternativen Ausführungsform der Erfindung ist die Mitnehmerplatte am Gleitring oder an der Gleitringaufnahme zwar drehbeweglich aber in vertikaler Richtung unbeweglich gelagert, wobei in diesem Fall die Druckplatte mit der Mitnehmerplatte in vertikaler Richtung beweglich verbunden ist. Bei dieser Ausführung bewegt sich beim Ausfahren der Hubeinheit die Druckplatte relativ zur Mitnehmerplatte in vertikaler Richtung.

Die Druckplatte und die Mitnehmerplatte weisen erfindungsgemäß jeweils eine mittige Öffnung auf, um ein Einbringen von Flüssigkeit in den Behälter sowie ein geregeltes Ableiten von Dampf aus dem Behälter zu ermöglichen.

Weitere Merkmale, Vorteile und Einzelheiten der Erfindung werden nun anhand der schematischen Zeichnung näher erläutert. Dabei zeigen
- Fig. 1: eine Ansicht einer erfindungsgemäßen Vorrichtung,

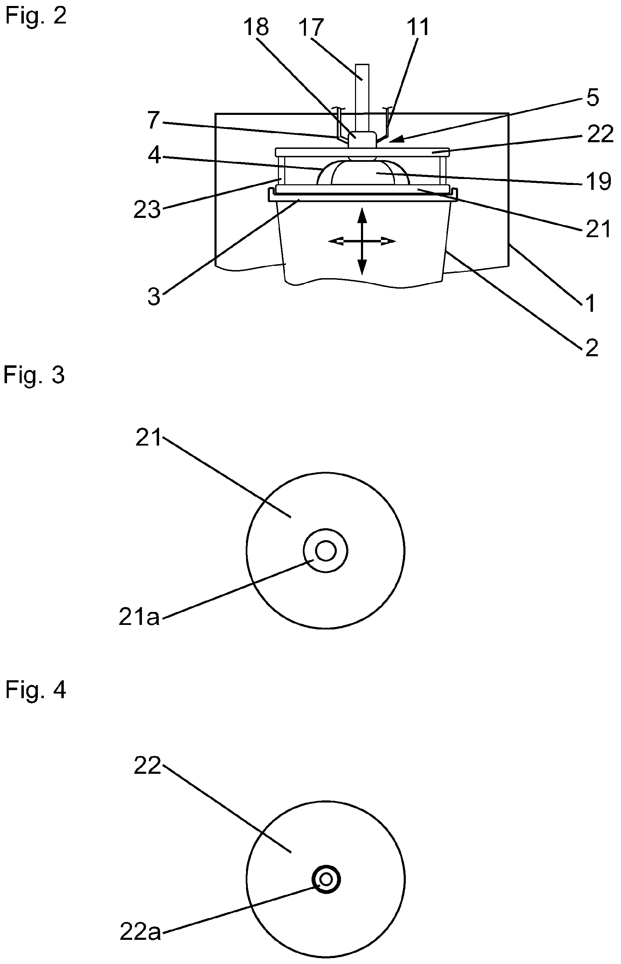

- Fig. 2: eine Ansicht einer Hubeinheit in ihrer Lage bei abgedichtetem Behälter,
- Fig. 3: eine Draufsicht auf eine Druckplatte und
- Fig. 4: eine Draufsicht auf eine Mitnehmerplatte.

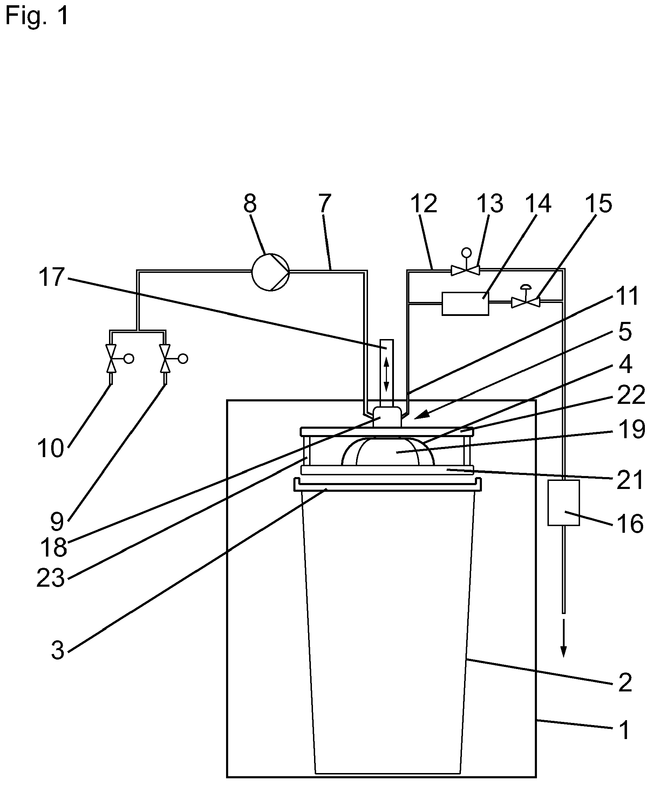

Die in Fig. 1 schematisch gezeigte Vorrichtung weist ein Mikrowellengerät mit nicht dargestellten Magnetronen zur Erzeugung hochfrequenter Mikrowellenstrahlung in einer Behandlungskammer 1 auf. Am Boden der Behandlungskammer 1 befindet sich ein nicht gezeigter Drehteller, auf welchem ein Behälter 2 positionierbar ist, in welchem das zu desinfizierende bzw. zu sterilisierende Gut, beispielsweise klinischer Abfall in einem Behälter, eingebracht ist. Der Behälter 2 ist mittels eines Deckels 3 verriegelbar, welcher mittig eine Öffnung aufweist, über welche eine Dichtungsglocke 4, die Bestandteil einer Hubeinheit 5 ist, stülpbar und auf der Oberseite des Deckels 3 aufsetzbar ist. Durch die mittige Öffnung im Deckel 3 ragt eine nicht dargestellte Düse in das Innere des Behälters 2, über welche zur Befeuchtung des Gutes im Behälter 2 Flüssigkeit in das Behälterinnere eingebracht werden kann. In die Hubeinheit 5 mündet eine mit der Düse verbundene Leitung 7, über welche mittels einer Pumpe 8, insbesondere einer Schlauchpumpe, von einem Wasseranschluss 9 Wasser und optional aus einem Behälter 10 ein Geruchsneutralisator zugeführt werden können. Die entsprechenden Mengen an Wasser und Geruchsneutralisator bzw. deren Mengenverhältnisse werden mittels regelbarer Ventile eingestellt. Die Hubeinheit 5 ist mit einer zweiten Leitung 11 verbunden, durch welche über das Innere der Hubeinheit 5 Dampf aus dem Behälter 2 ableitbar ist. In der Leitung 11 befinden sich eine Temperaturmesseinrichtung 14 sowie ein Regelventil 15. Bevor das in der Leitung 11 abgeleitete Kondensat nach außen abgeführt wird, durchläuft es einen Wärmetauscher 16, mittels welchem das zur Befeuchtung des Desinfektionsgebindes zugeführte Wasser vorgewärmt werden kann. In einem Bypass 12 der Leitung 11 befindet sich ein Dampfableitventil 13.

Wie beispielsweise Fig. 1 zeigt, weist die Hubeinheit 5 einen Hubmotor 17, eine Gleitringaufnahme 18 und einen mit der Gleitringaufnahme 18 fest verbundenen, etwa kuppelförmigen Gleitring 19 auf. Der Hubmotor 17, die Gleitringaufnahme 18 und der Gleitring 19 sind miteinander fest verbunden. Den kuppelförmigen Gleitring 19 umgibt außenseitig die Dichtungsglocke 4, die an der Gleitringaufnahme 18 mittels einer Gleitdichtung bzw. einem Gleitlager drehbar, aber flüssigkeits- und dampfdicht gelagert ist. Die kreisförmigen Dichtflächen der Dichtungsglocke 4 und des Gleitringes 19 befinden sich in der gleichen, horizontal verlaufenden Ebene. Auf der Hubeinheit 5 sitzt eine Anordnung aus zwei übereinander sowie parallel zueinander und horizontal verlaufenden Platten, einer Druckplatte 21 und einer Mitnehmerplatte 22, wobei die Druckplatte 21 die weiter unten befindliche Platte ist und die beiden Platten 21, 22 miteinander über eine bestimmte Anzahl von Mitnehmern 23 bzw. Mitnehmerstegen fest verbunden sind. Sowohl die Mitnehmerplatte 22 als auch die Druckplatte 21 sind vorzugsweise kreisförmige Platten, die Druckplatte 21 ist bezüglich ihres Außendurchmessers an den Durchmesser des Deckels 3 derart angepasst, dass sie flächig auf dem Deckel 3, diesen möglichst komplett bedeckend, positionierbar ist. Der durch die Höhe der Mitnehmer 23 bestimmte gegenseitige Abstand der Platten 21 und 22 ist geringfügig größer als die vertikale Erstreckung des Gleitringes 19 und der Dichtungsglocke 4. Die Mitnehmerplatte 22 weist eine mittige Öffnung 22a auf, mittels welcher sie über der Gleitringaufnahme 18 positioniert ist und gegenüber dieser in vertikaler Richtung bewegbar ist. In der in Fig. 1 gezeigten Ausgangsposition, in welcher der Hubmotor 17 eingefahren ist und die Hubeinheit 5 sich in ihrer oberen Position befindet, liegt die Mitnehmerplatte 22 außen auf dem Gleitring 19 bzw. der Dichtungsglocke 4 auf, die Druckplatte 21, welche eine mittige Öffnung 21 a zum Durchführen der Düse zum Einbringen der Flüssigkeit in den Behälter 2 aufweist, befindet sich in einem geringen Abstand unterhalb der Dichtflächen der Dichtungsglocke 4 und des Gleitringes 19.

Von der Gleitringaufnahme 18 geht außenseitig sowohl die Kondensatleitung 11 als auch die mit der Düse verbundene Leitung 7 ab.

Die Befeuchtung des zu sterilisierenden/desinfizierenden, im Behälter 2 eingebrachten Gutes erfolgt vollautomatisch durch entsprechende Zufuhr von Flüssigkeit vor dem Aufheizvorgang. Die Flüssigkeitsmenge zur optimalen Befeuchtung kann beispielsweise auf die im österreichischen Patent Nr. 403007 beschriebene Weise festgestellt werden, indem der Behälter 2 auf dem als Wiegeteller ausgeführten Drehteller positioniert wird. Aus dem ermittelten Gesamtgewicht kann nach einem bestimmten Algorithmus die erforderliche Flüssigkeitsmenge ermittelt und zugegeben werden.

Zum Betrieb der erfindungsgemäßen Vorrichtung wird der mit dem Deckel 3 fest verschlossene Behälter 2 mitsamt dem bereits im Behälter 2 befindlichen, zu sterilisierenden bzw. zu desinfizierenden Gut in die Behandlungskammer 1 eingebracht, nach dem Schließen der Behandlungskammer 1 wird durch den Hubmotor 17 die Hubeinheit 5 in vertikaler Richtung nach unten verfahren. Dabei wird die Druckplatte 21 flächig auf der Außenseite des Deckels 3 aufgesetzt, die Dichtungsglocke 4 und der Gleitring 19 auf der Außenseite der Druckplatte 21. Die Mitnehmerplatte 22 gleitet dabei gegenüber der Gleitringaufnahme 18 etwas nach oben. Nach dem Starten des Behandlungszyklus wird die ermittelte Flüssigkeitsmenge zur Befeuchtung des Gebindes durch die Düse in den Behälter 2 eingespritzt, wobei, wie oben erwähnt, Wasser oder Wasser mit einer bestimmten Menge an Geruchsneutralisator verwendet wird. Die Erhitzung erfolgt durch die hochfrequente Mikrowellenstrahlung der Magnetrone. Die Aufheizzeit ist abhängig von der Masse des Gutes im Behälter 2 und dauert einige Minuten.

Nach Erreichen der Kochtemperatur wird automatisch auf die vorgegebene Haltezeit des Behandlungszyklus umgeschaltet. Ist diese Haltezeit von beispielsweise 20 bis 30 Minuten abgelaufen, kühlt das Gerät noch einige Minuten, insbesondere fünf Minuten, ab. Der Temperatursollwert wird durch eine kontrollierte Dampfableitung mit Hilfe des Regelventils 15 und des Dampfableitventils 13 erreicht. Dabei wird die Temperatur des Dampfes vor dem Regelventil 15 mittels der Temperaturmesseinrichtung 14 erfasst. Um nach dem Desinfektionsprozess den verbleibenden Dampfdruck rasch abbauen zu können, wird das Dampfableitventil 13 geöffnet.

Während des Aufheizvorganges wird der Behälter 2 mittels des nicht dargestellten Drehtellers in Drehbewegung versetzt. Die auf der Druckplatte 21 fest aufsitzende Dichtungsglocke 4 folgt mitsamt der Druckplatte 21 und der Mitnehmerplatte 22 der Drehbewegung, der Gleitring 19 verbleibt ortsfest und gleitet auf der Deckeloberseite. Die zwischen der Gleitringaufnahme 18 und der Dichtungsglocke 4 eingebaute Dichtung sorgt für eine Abdichtung nach außen. Über die Druckplatte 21 wird der von innen auf den Deckel 3 wirkende Dampfdruck gleichmäßig verteilt, der Gleitring 19 nimmt Druck von der Druckplatte 21 auf, sodass während des gesamten Behandlungszyklus der Behälter 2 nach außen abgedichtet bleibt.

Bei einer alternativen, nicht dargestellten Ausführungsform der Erfindung ist die Mitnehmerplatte am Gleitring oder der Gleitringaufnahme zwar drehbeweglich aber in vertikaler Richtung unbeweglich gelagert. Die Druckplatte ist in diesem Fall mit der Mitnehmerplatte in vertikaler Richtung beweglich verbunden.

### Bezugsziffernliste

- 1: Behandlungskammer
- 2: Behälter
- 3: Deckel
- 4: Dichtungsglocke
- 5: Hubeinheit
- 7: Leitung
- 8: Pumpe
- 9: Wasseranschluss
- 10: Behälter
- 11: Leitung
- 12: Bypass
- 13: Dampfableitventil
- 14: Temperaturmesseinrichtung
- 15: Regelventil
- 16: Wärmetauscher
- 17: Hubmotor
- 18: Gleitringaufnahme
- 19: Gleitring
- 21: Druckplatte
- 21a: Öffnung
- 22: Mitnehmerplatte
- 22a: Öffnung
- 23: Mitnehmer

## Patentansprüche

1. Vorrichtung zum Erhitzen, Desinfizieren und/oder Sterilisieren eines Gutes unter Überdruck durch Erhitzen des Gutes mittels Mikrowellen,
mit einem in einer Behandlungskammer (1) auf einem Drehteller positionierbaren Behälter (2), in welchem sich das zu behandelnde Gut befindet und welcher mit einem Deckel (3) verriegelbar ist, welcher mit einer Öffnung versehen ist, durch welche Flüssigkeit in das Behälterinnere einbringbar ist,
und mit einer Hubeinheit (5) mit einem Hubmotor (17), einer Gleitringaufnahme (18) und einem auf dem Deckel (3) aufsetzbaren kuppelförmigen Gleitring (19), wobei der Hubmotor (17), die Gleitringaufnahme (18) und der Gleitring (19) miteinander fest verbunden sind,
sowie mit einer gegenüber der Gleitringaufnahme (18) abgedichteten jedoch drehbaren, auf dem Deckel (3) aufsetzbaren Dichtungsglocke (4), welche über den Gleitring(18) gestülpt ist,
**dadurch gekennzeichnet,**
**dass** auf der Hubeinheit (5) eine Anordnung aus einer an die Außenabmessungen des Deckels (3) angepassten Druckplatte (21) und einer oberhalb der Druckplatte (21) und von dieser in vertikaler Richtung beabstandeten, mit der Druckplatte (21) verbundenen Mitnehmerplatte (22) positioniert ist, wobei die Mitnehmerplatte (22) gegenüber dem Gleitring (19) oder der Gleitringaufnahme (18) drehbar ist und die Druckplatte (21) in vertikaler Richtung bewegbar ist, wobei bei ausgefahrener Hubeinheit (5) die Druckplatte (21) von der Dichtungsglocke (4) und dem Gleitring (19) auf den Behälterdeckel (3) gedrückt wird.

2. Vorrichtung nach Anspruch 1, **dadurch gekennzeichnet, dass** die Druckplatte (21) und die Mitnehmerplatte (22) miteinander fest verbunden sind, wobei die Mitnehmerplatte (22) gegenüber der Gleitringaufnahme (18) drehbar und in vertikaler Richtung bewegbar ist und in der eingefahrenen Position der Hubeinheit (5) auf dem Gleitring (19) bzw. der Dichtungsglocke (4) aufliegt.

3. Vorrichtung nach Anspruch 2, **dadurch gekennzeichnet, dass** die Druckplatte (21) und die Mitnehmerplatte (22) über stegartig ausgebildete Mitnehmer (23) miteinander fest verbunden sind.

4. Vorrichtung nach Anspruch 1, **dadurch gekennzeichnet, dass** die Mitnehmerplatte am Gleitring oder an der Gleitringaufnahme zwar drehbeweglich aber in vertikaler Richtung unbeweglich gelagert ist, wobei die Druckplatte mit der Mitnehmerplatte in vertikaler Richtung beweglich verbunden ist.

5. Vorrichtung nach einem der Ansprüche 1 bis 4, **dadurch gekennzeichnet, dass** die Druckplatte (21) und die Mitnehmerplatte (22) jeweils eine mittige Öffnung (21a, 22a) aufweisen.
